Europäisches Patentamt

(19) European Patent Office

Office européen des brevets

(11) Publication number: **0 106 282**
**A2**

(12) # EUROPEAN PATENT APPLICATION

(21) Application number: 83110012.8

(22) Date of filing: 06.10.83

(51) Int. Cl.³: **C 07 C 103/52**

(30) Priority: 08.10.82 JP 178181/82

(43) Date of publication of application:
25.04.84 Bulletin 84/17

(84) Designated Contracting States:
BE CH DE FR IT LI NL

(71) Applicant: Shionogi & Co., Ltd.

Fukushima-ku Osaka, 553(JP)

(72) Inventor: Kikkawa, Ikuo
18-21, Tsurunoso Takarazuka-shi
Hyogo(JP)

(74) Representative: Vossius Vossius Tauchner Heunemann Rauh
Siebertstrasse 4 P.O. Box 86 07 67
D-8000 München 86(DE)

(54) Production of caerulein intermediate.

(57) A process for producing a decapeptide of the formula:

H-Pyr-Gln-Asp-Tyr-Thr(Ac)-Gly-Trp-Met-Asp-Phe-NH₂     (I)

being useful as an intermediate in the production of caerulein as diagnostic agent or psychotropic agent, which comprises subjecting the hexapeptide represented by the formula:

H-Pyr-Gln-Asp-Tyr-Thr(Ac)-Gly-NHNH₂     (II)

to azide forming reaction at the C-terminal and then to reaction with the tetrapeptide represented by the formula:

H-Trp-Met-Asp-Phe-NH₂     (III)

VOSSIUS · VOSSIUS · TAUCHNER · HEUNEMANN · RAUH

PATENTANWÄLTE

SIEBERTSTRASSE 4 · 8000 MÜNCHEN 86 · PHONE: (089) 47 40 75
CABLE: BENZOLPATENT MÜNCHEN · TELEX 5-29 453 VOPAT D

0106282

Shionogi & Co., Ltd.
Our Ref: S 627 EP

6. Okt. 1983

## Production of Caerulein Intermediate

### Background of the Invention

The present invention relates to a novel process for producing a decapeptide, as an intermediate in the production of caerulein, having the following structural formula;

$$H-Pyr-Gln-Asp-\overset{\overset{\textstyle SO_3H}{|}}{Tyr}-Thr-Gly-Trp-Met-Asp-Phe-NH_2$$

which was isolated from the extract of the skin of Hyla-caerulea, a kind of frog in Australia, by Anastasi et al.

It is known that caerulein has activities analogous to those of the hormones of the digestive organ such as gastrin, pancreozaimine and the like. Therefore, it has been used now as an agent for diagnosis of the drug for treating the progressing paralytic ileus. Lately, it has been elucidated that caerulein is effective in treatment of a sort of mental deseases, particularlly, schizophrenia, manic- or depressive psychosis, senile dementia and the like. Therefore, the research and development of caerulein has been focused on its possibility as psycotropic drugs.

Some of the processes for producing caerulein had been disclosed, for example, in Japanese Patent Publication No. 44-25079, Japanese Unexamined Patent

- 1 -

Publication Nos. 54-61170, 54-63072 and 55-100348.
The most important step in the production of caerulein
is sulfonation of the tyrosine residue which is
located at the 7th from the C-terminal of the decapeptide. In the prior art as mentioned above, the said
sulfonation reaction is achieved with a pyridine/sul-
furic anhydride $(SO_3)$ in anhydrous dimethylformamide
or in an aqueous solvent in a prefixed pH-range
(pH 8.5-12.5).

The present inventors developed a novel improved
process for the sulfonation with sulfuric anhydride,
whereby the treatment of the reaction product after
the reaction completion is easy, which comprises using
the copolymer of vinylpyridine with divinylbenzen instead of pridine bases (Japanese Patent Application
No. 57-90927). The decapeptide (I) prepared in this
invention is an important intermediate in producing
caerulein because it can readily be converted into
caerulein by sulfonation of the 7th amino acid tyrosine
from the C-terminal and subsequent deacetylation of
the 6th amino acid threonine.

Summary of the Invention

The present invention relates to a novel process
for producing a caerulein intermediate decapeptide
represented by the formula:

-3-

H-Pyr-Gln-Asp-Tyr-Thr(Ac)-Gly-Trp-Met-Asp-Phe-NH$_2$ (I)

which comprises subjecting the hexapeptide represented

by the formula:

H-Pyr-Gln-Asp-Tyr-Thr(Ac)-Gly-NHNH$_2$ (II)

to azide forming reaction at the C-terminal and then to reaction with the tetrapeptide represented by the

formula:

H-Trp-Met-Asp-Phe-NH$_2$ (III)

[wherein Ac means acetyl].

Description of the Preferred Enbodiment

The objective compound (I) in this invention can be produced by condensation of the hexapeptide of the above formula (II) with the tetrapeptide of the formula (III) according to the conventional method. For example, the C-terminal hydrazide of the hexapeptide (II) is allowed to react with nitrous acid or derivatives thereof to give the corresponding C-terminal azide compound (IV):

H-Pyr-Gln-Asp-Tyr-Thr(Ac)-Gly-N$_3$  (IV)

which is then allowed to react with the tetrapeptide (III). The formation of the azide compound (IV) from hexapeptide (II) may be carried out under the known reaction conditions for azide formation; for example, the hexapeptide (II) is dissolved in a polar solvent such as dimethylformamide, dimethylsulfoxide and so on, which is acidified with hydrochloric acid, and to which a nitrite such as sodium nitrite or isoamyl nitrite may be added at a low temperature (about -10°C - 10°C). The resulting azide compound (IV) without isolation from the reaction mixture is directly subjected to reaction with the tetrapeptide (III). In more detail, the reaction mixture containing the azide compound (IV) is basified with addition of an organic base such as triethylamine, tri-n-butylamine and the like, to which a dimethylformamide or dimethylsulfoxide solutions of tetrapeptide (III) is added at a low temperature (about -50 - -20°C, preferably, -30 - -25°C) and the resulting solution is allowed to stand at the same temperature for a few days. The objective product (I) may be isolated as a purified compound by means of conventional isolation technique, for example, evaporation of the solvent, reprecipitation, washing with solvents, drying and the like.

The starting hexapeptide (II) in this invention is produced from the C-terminal glycine derivative through the six steps as follows.

The threonine derivative represented by the formula:

BOC-Thr(Ac)OH    (VI)

or its reactive derivatives are subjected to reaction with glycylhydrazine derivatives represented by the formula:

H-Gly-NHNH-Z    (V)

to give the dipeptide represented by the formula:

BOC-Thr(Ac)-Gly-NHNH-Z    (VII)

which after elimination of the protecting group at the N-terminal of the dipeptide is allowed to react with the tyrosine derivative represented by the formula:

BOC-Try(Bzl)OH   (VIII)

or its reactive derivatives to give the tripeptide represented by the formula:

BOC-Tyr(Bzl)-Thr(Ac)-Gly-NHNH-Z   (IX)

which after elimination of the protecting group at the N-terminal is allowed to react with the aspartic acid derivatives represented by the formula:

BOC-Asp(OBzl)OH   (X)

or its reactive derivative to give the tetrapeptide represented by the formula:

BOC-Asp(OBzl)-Tyr(Bzl)-Thr(Ac)-Gly-NHNH-Z   (XI)

which after elimination of the protecting group at the N-terminal is allowed to react with the glutamine derivative represented by the formula:

BOC-Gln-OH   (XII)

or its reactive derivatives to give the pentapeptide represented by the formula:

BOC-Gln-Asp(OBzl)-Tyr(Bzl)-Thr(Ac)-Gly-NHNH-Z   (XIII)

which after elimination of the protecting group at the N-terminal is allowed to react with the pyroglutamic acid derivatives represented by the formula:

Z-Pyr-OH   (XIV)

or its reactive derivatives to give the hexapeptide represented by the formula:

Z-Pyr-Gln-Asp(OBzl)-Tyr(Bzl)-Thr(Ac)-Gly-NHNH-Z   (XV)

-7-

which is deprotected to give the objective compound (II)
[wherein the symbols BOC, Z and Bzl mean t-butyloxycarbonyl,
benzyloxycarbonyl and benzyl, respectively].

The hexapeptide (II) may also be produced as shown below.

The tetrapeptide represented by the formula:

H-Pyr-Gln-Asp-Tyr-NHNH$_2$    (XVI)

is condensed with the dipeptide represented by the formula:

H-Thr(Ac)-Gly-NHNH-Z    (XVII)

in the known method, followed by deprotection of the
hydrazide.

On the other hand, another starting material tetrapeptide (III) is a known compound disclosed in J. Chem.
Soc., (c) 555 (1966), which may be used as its acid
addition salt such as the trifluoroacetate.

The present invention is explained in more detail
according to the following Preparations and Example. The
symbols used in the structural formulas in Preparations and
Example have the same meanings as mentioned in the formulas
(I) - (XVII).

Preparation of starting material (1)

1)        BOC-Gly-NHNH-Z  :

To a solution of 25g (0.143 mol) of t-butyloxycarbonylglycine in 300ml of tetrahydrofuran are added
16.46g (1.0 mol eq.) of N-hydroxysuccinimide and 2.95g
(1.0 mol eq.) of dicyclohexylcarbodiimide (DCC) under
ice-cooling and the mixture is allowed to stand overnight.

Then, the precipitated dicyclohexylurea is filtered off and the filtrate is condensed under reduced pressure and crystallized from ethyl acetate/petroleum ether to give 33.09g (in 85.0% yield) of t-butyloxycarbonylglycine・N-succinimide ester. mp. 160-162°C

The N-succinimide ester is dissolved in a mixture of dimethylformamide (200ml) and methylene chloride ( 200ml) and a solution of 30.58g (0.184mol) of benzyloxy-carbonylhidradine in 50 ml of methylene chloride is added thereto under stirring and ice-cooling, and the mixture is allowed to stand overnight under ice-cooling. The reaction mixture is condensed under reduced pressure and the residue is dissolved in ethyl acetate, then washed with chilled diluted hydrochloric acid, water, a chilled aqueous solution of sodium hydrogencarbonate and·water, successively, and condensed under reduced pressure; the residue is recrystallized from ether・petroleum ether to give 56.95g (in 95.71% yield) of N-(t-butyloxycarbonylglycyl)-N'-benzyloxycarbonylhydra-zine as pillars. m.p. 85-87°C.

Anal. Calcd.(%) for $C_{15} H_{21} N_3 O_5$ :

C, 55.72 ; H, 6.55 ; N, 13.00

Found (%) :

C, 55.61 ; H, 6.50 ; N, 13.30

2)   BOC-Thr(Ac)-Gly-NHNH-Z   (VII) :

To a   solution of 56.95g (0.176 mol) of the above
product in 300ml of acetic acid is added 300ml (3 mol
eq.) of 1.76N-dry hydrochloric acid/acetic acid, and
after one hour at a room temperature, the mixture is
condensed under reduced pressure.   To the residue·
is added 300ml of toluene, from which most of the acetic
acid is removed by azeotropic distillation.   Then,
500ml of ether is added to the residue and the suspension is stirred to give 43.3g (in 94.75% yield) of N-
glycyl-N'-benzyloxycarbonylhydrazine hydrochloride as ·
crystals.   m.p. 177-180°C.

Anal. Calcd. (%) for $C_{10}H_{13}N_3O_3 \cdot HCl$ :

C, 46.25 ; H, 5.43 ; N, 16.18 ; Cl, 13.65
Found (%) :

C, 46.12 ;   H, 5.34 ; N, 15.98 ; Cl, 13.61

In 358ml of dimethylformamide is dissolved 35.84g
(0.138 mol) of the above crystal, to which 329ml (0.1
mol eq.) of tri-n-butylamine and 49.5g (0.138 mol)
of t-butyloxycarbonyl-O-acetylthreonine·N-succinimide
ester [Experientia, 23, 700 (1967)] are added under
ice-cooling, and the mixture is allowed to stand at
the same temperature for 23 hours.   The residue dissolved in ethyl acetate is washed with chilled diluted
hydrochloric acid, water, a chilled aqueous solution of sodium hydrogencarbonate and water successively, condensed under reduced pressure and then crystallized from 300ml of petroleum ether to give 60.25g (in 93.58% yield) of N-(t-butyloxycarbonyl-0-acetyl-L-threonyl-glycyl)-N'-benzyloxycarbonylhydrazine (VII) as crystals. m.p. 54 - 65°C.  $[\alpha]_D^{22}$ +17.0±0.6° (c, 1.01, methanol).

Anal. Calcd. (%) for $C_{21}H_{30}N_4O_8$ :

    C, 54.07 ; H, 6.48 ; N, 12.01

Found (%) :

    C, 54.55 ; H, 6.80 ; N, 11.52

3)    BOC-Tyr(Bzl)-Thr(Ac)-Gly-NHNH-Z    (IX) :

The above product (VII) (32.42g) [0.0695 mol] is subjected to elimination of the protecting group under the same conditions as in the aforementioned steps to give 26.99g (in 96.39% yield) of N-(0-acetyl-L-threonyl-glycyl)-N'-benzyloxycarbonylhydrazine hydrochloride as crystals.  m.p. 64 - 100°C.  $[\alpha]_D^{22}$ +6.0±0.5° (c, 1.008, methanol)

Anal. Calcd. (%) for $C_{16}H_{22}N_4O_6 \cdot HCl$ :

    C, 47.71 ; H, 5.76 ; N, 13.91 ; Cl, 8.8C

Found (%) :

    C, 47.81 ; H, 6.28 ; N, 13.09 ; Cl, 8.99

To a solution of 26.74g (0.0664 mol) of the above product in  270ml of methylformamide are added 12.3g (1.0 mol eq.) of tri-n-butylamine and 31.1g (1.0 mol eq.) of t-butyloxycarbonyl-O-benzyl-L-tyrosine·N-succinimide ester under stirring and ice-cooling, and the mixture is allowed to stand overnight.   The reaction mixture is condensed under reduced pressure and the residue is dissolved in ethyl acetate, then washed with a chilled diluted hydrochloric acid, water, a chilled aqueous solution of sodium hydrogencarbonate and water, successively, and condensed under reduced pressure.   The residue is crystallized from 350ml of ether to give 31.8g (in 66.5% yield) of N-(t-butyloxycarbonyl-O-benzyl-L-tyrosyl-O-acetyl-L-threonyl-glycyl)-N'-benzyloxycarbonylhydrazine (IX) as crystals.   m.p. 167-169°C.   $[\alpha]_D^{22}$ +13.6±0.5°  (c, 1.019, methanol)

Anal. Calcd. (%) for $C_{37}H_{45}N_5O_{10}$ :

    C, 61.74 ; H, 6.30 ; N, 9.73

Found (%) :

    C, 60.99 ; H, 6.22 ; N, 9.54

4)    BOC-Asp(OBzl)-Tyr(Bzl)-Thr(Ac)-Gly-NHNH-Z    (XI) :

Under the same conditions as in the above steps, 21.05g (29.2 m mol) of the above product (IX) is subjected to elimination of the protecting group to give 18.74g (in 97.6% yield) of N-(O-benzyl-L-tyrosyl-O-acetyl-L-threonyl-glycyl)-N'-benzyloxycarbonylhydrazine hydrochloride.   m.p. 120-128°C    $[\alpha]_D^{23}$  +21.3±0.6° (c, 1.0, methanol)

Anal. Calcd. (%) for $C_{32}H_{37}N_5O_8 \cdot HCl \cdot 1/2H_2O$ :

   C, 57.78 ; H, 5.91 ; N, 10.54 ; Cl, 5.33 ; $H_2O$, 1.35

Found (%) :

   C, 57.63 ; H, 5.86 ; N, 10.54 ; Cl, 5.13 ; $H_2O$, 0.69

To a solution of 18.58g (28.3m mol) of the above·
product in 186ml of dimethylformamide are added 6.7ml
(28.3m mol) of tri-n-butylamine and 11.9g (28.3m mol)
of N-succinimide t-butyloxycarbonyl-β-benzyl-L-aspartate,
and the mixture is allowed to stand overnight. The
reaction mixture is condensed under reduced pressure,
and the residue is solidified with a mixture of ethyl
acetate and ether. The resultant solid is collected
by filtration, washed with a chilled diluted hydrochloric acid, water, a chilled aqueous solution of
sodium hydrogencarbonate and·water, successively, then
suspended in ethyl acetate, and collected by filtration
to give 24.19g (in 92.0% yield) of N-(t-butyloxycarbonyl-β-benzyl-L-aspartyl-0-benzyl-L-tyrosyl-0-acetyl-
L-threonyl-glycyl)-N'-benzyloxycarbonyl hydrazine (XI).
m.p. 157 - 165°C. $[\alpha]_D^{22}$ +1.6±0.4° (c, 1.001, methanol)

Anal. Calcd. (%) for $C_{48}H_{56}N_6O_{13}$ :

   C, 62.33 ; H, 6.10 ; N, 9.09

Found (%) :

   C, 61.59 ; H, 5.97 ; N, 9.16

5) BOC-Gln-Asp(OBzl)-Tyr(Bzl)-Thr(Ac)-Gly-NHNH-Z  (XIII)

Under the same conditions as mentioned in the above steps, 24.08g(25.92m mol) of the above product (XI) is subjected to elimination of the protecting group to give 22.3g (in 100% yield) of N-(β-benzyl-L-aspartyl-O-benzyl-L-tyrosyl-O-acetyl-L-threonyl-glycyl)-N'-benzyloxycarbonylhydrazine hydrochloride.   $[\alpha]_D^{22}$ +19.0 ±0.6° (c, 1.007, methanol)

Anal. Calcd. (%) for  $C_{43}H_{48}N_6O_{11}$ HCl $H_2O$:

C, 58.73 ; H, 5.85 ; N, 9.56 ; Cl, 4.03 ;

$H_2O$, 2.05

Found (%):

C, 58.65 ; H, 5.77 ; N, 9.78 ; Cl, 3.99 ;

$H_2O$, 1.90

To a solution of 22.13g (25.7m mol) of the above product in 220ml of dimethylformamide are added 6.1ml (1.0 mol eq.) of tri-n-butylamine and 8.82g (25.7m mol) of N-succinimide t-butyloxycarbonyl-L-glutamate under ice-cooling and the mixture is allowed to stand overnight.   The reaction mixture is condensed under reduced pressure, and ethyl acetate is added to the residue and stirred.   The precipitated solid is collected by filtration, then washed with a chilled diluted hydrochloric acid, water, a chilled aqueous solution of sodium hydrogencarbonate and water, successively, suspended in ethyl acetate and collected by filtration to give 23.85g (in 88.0% yield) of N-(t-butyloxycarbonyl-L-glutaminyl-$\beta$-benzyl-L-aspartyl-0-benzyl-L-tyrosyl-0-acetyl-L-threonyl-glycyl)-N'-benzyloxycarbonylhydrazine (XIII).   m.p. 219-220°C.

$[\alpha]_D^{22}$ -2.1±0.4°   (c, 1.007, dimethylformamide)

Anal. Calcd. (%) for $C_{53}H_{64}N_8O_{15}$ 1/2 $H_2O$ :

   C, 59.93 ; H, 6.17 ; N, 10.55 ; $H_2O$, 0.85

Found (%) :

   C, 59.87 ; H, 6.10 ; N, 10.87 ; $H_2O$, 0.75

6)   Z-Pyr-Gln-Asp(OBzl)-Tyr(Bzl)-Thr(Ac)-Gly-NHNH-Z   (XV):

Under the same conditions as in the above steps, 23.69g (22.49m mol) of the above product (XIII) is subjected to elimination of the protecting group to give 22.41g (in 100% yield) of N-(L-glutaminyl-$\beta$-benzyl-L-aspartyl-0-benzyl-L-tyrosyl-0-acetyl-L-threonyl-glycyl)-N'-benzyloxycarbonylhydrazine hydrochloride.   $[\alpha]_D^{23}$ +4.4±0.4°   (c, 1.010, dimethylformamide)

Anal. Calcd. (%) for  $C_{48}H_{56}N_8O_{13}$ HCl $H_2O$ :

   C, 57.22 ; H, 5.90 ; N, 11.12 ; Cl, 3.62 ;

   $H_2O$, 1.79

Found (%) :

   C, 56.85 ; H, 5.98 ; N, 11.00 ; Cl, 3.48 ;

   $H_2O$, 1.19

To a solution of 22.29g (22.49m mol) of the above product in 220ml of dimethylformamide are added 5.4ml (22.49m mol) of tri-n-butylamine and 8.1g (22.49m mol) of N-succinimide benzyloxycarbonyl-pyroglutamate and the mixture is allowed to stand overnight under ice-cooling.  The reaction mixture is condensed under reduced pressure, and 170ml of ethyl acetate is added to the residue.  The precipitated solid is collected by filtration, then washed a chilled diluted hydrochloric acid, water, a chilled aqueous solution of sodium hydrogencarbonate and water, successively, suspended in ethyl acetate and collected by filtration to give 24.57g (in 91.3% yield) of N-(benzyloxycarbonyl-L-pyroglutamyl-L-glutaminyl-β-benzyl-L-aspartyl-O-benzyl-L-tyrosyl-O-acetyl-L-threonyl-glycyl)-N'-benzyloxycarbonylhydrazine (XV).   $[\alpha]_D^{23}$ -7.0±0.5° (c, 1.008, dimethylformamide)

Anal. Calcd. (%) for $C_{61}H_{67}N_9O_{17} \cdot 1/2\ H_2O$ :

C, 60.69 ; H, 5.68 ; N, 10.44 ; $H_2O$, 0.74

Found (%) :

C, 59.97 ; H, 5.84 ; N, 11.00 ; $H_2O$, 0.76

7)  H-Pyr-Gln-Asp-Tyr-Thr(Ac)-Gly-NHNH$_2$ HCl

as the hydrochloride of the compound (II) :

To a solution of 24.0g (20m mol) of the above product (XV) in 360ml of dimethylformamide are added 22.7ml (2 mol eq.) of 1.76N-dry hydrochloric acid/acetic acid and 53.2ml of palladium black, and then the mixture is stirred under hydrogen atmosphere for 6 hours. The palladium catalyst is removed by filtration and washed with dimethylformamide; the filtrate and washings are combined and evaporated under reduced pressure. The residue is solidified from a mixture of methanol and ether to give 16.9g (in 100% yield) of L-pyroglutamyl-L-glutaminyl-L-aspartyl-L-tyrosyl-O-acetyl-L-threonyl-glycylhydrazide hydrochloride. $[\alpha]_D^{22.5}$ -7.2±0.5° (c, 1.016, dimethylformamide)

Anal. Calcd. (%) for $C_{31}H_{43}N_9O_{13} \cdot HCl \cdot H_2O$ :

C, 46.30 ; H, 5.77 ; N, 15.68 ; Cl, 4.53

$H_2O$, 2.24

Found (%) :

C, 45.58 ; H, 6.30 ; N, 15.50 ; Cl, 4.51

$H_2O$, 2.26

Example

H-Pyr-Gln-Asp-Tyr-Thr(Ac)-Gly-Trp-Met-Asp-Phe-NH$_2$ (I) :

To a solution of 15.6g (16.57m mol x 1.2) of the above product [the hydrochloride of (II)] in 312ml of dimethylformamide, which is cooled to -35°C (bath temperature), are added 12.22ml (1.2 × 3 mol eq.) of dry hydrochloric acid /dioxane (4.9 mol/l) and 3.19ml (1.2 × 1.2 mol eq.) of isoamyl nitrite, and the mixture is stirred for 20 minutes. The reaction mixture is then cooled

- 17 -

to about -45°C, and 22.89ml (1.2 x 4 + 1mol eq.) of tri-n-butylamine is dropwise added thereto; and then a solution of 11.93g (19.57m mol) of L-tryptophyl-L-methyonyl-L-aspartyl-L-phenylaianinamide trifluoracetate in 72ml of dimethylformamide is added thereto at the same temperature, and the mixture is allowed to stand at a temperature of -30°C- -25°C for 3 days, during which time tri-n-butylamine (11.43ml as a total amount) is occasionally added dropwise in order to keep the reaction mixture at pH 6 - 7.  The reaction mixture is neutralized with 3.6ml of acetic acid and condensed under reduced pressure.  The residue is suspended in 480ml of 0.3N-acetic acid, and the precipitating solid is collected by filtration, washed with water, methanol and ether, successively, and then dried on phosphorus pentoxide under reduced pressure at room temperature for 20 hours to give 17.03g (in 78% yield) of the objective product L-pyroglutamyl-L-glutaminyl-L-aspartyl- L- tyrosyl-0-acetyl-L-threonyl-glycyl-L-tryptophyl-L-methionyl-L-aspartyl-L-phenyl-alaninamide (I).

$[\alpha]_D^{22}$ -20.4±1.2° (c, 0.508, dimethylformamide)

Anal. Calcd. (%) for $C_{60}H_{75}N_{13}O_{19}S \cdot 2H_2O$ :

C, 53.37 ; H, 5.80 ; N, 13.48 ; S, 2.37 ;

$H_2O$, 2.67

Found (%):

C, 52.68 ; H, 5.93 ; N, 13.27 ; S, 2.42 ;

$H_2O$, 3.34

Preparation of starting material (2)

1) H-Pyr-Gln-Asp-Tyr-Thr(Ac)-Gly-NHNH-Z :

To a solution of 2.69g of L-pyroglutamyl-L-glu-taminyl-L-aspartyl-L-tyrosylhydrazide in a mixture of 7ml of dimethylsulfoxide and 21ml of dimethylformamide are added 3.81ml of 4.88M hydrochloric acid/acetic acid and 0.55ml of isoamyl nitrite, and the mixture is allowed to react for 20 minutes at a temperature of -23°C- -30°C. Then, to this reaction mixture are added a solution of 1.0g of N-(L-threonyl-glycyl)-N'-benzyloxycarbonylhydrazine in 6ml of dimethylform-amide and 0.3ml of tri-n-butylamine, and then the mixture is allowed to react at 4°C for 16 hours. The reaction mixture is worked up in the same manner as in Example (1) for preparing the starting material to give 0.92g (in 42.2% yield) of N-(L-pyroglutamyl-L-glutaminyl-L-aspartyl-L-tyrosyl-0-acetyl-L-threonyl-glycyl)-N'-benzyloxycarbonylhydrazine. m.p. 207 - 208°C (decomposition). $[\alpha]_D^{23}$ -5.0±0.5° (c, 1.0, dimethyl-formamide). Anal. calcd. (%) for $C_{39}H_{49}N_9O_{15}\cdot1/2H_2O$ :

C, 52.46 ; H, 5.64 ; N, 14.12 ; $H_2O$, 1.01

Found (%):

C, 51.46 ; H, 5.66 ; N, 14.54 ; $H_2O$, 1.12

2)   H-Pyr-Gln-Asp-Tyr-Thr(Ac)-Gly-NHNH$_2$·HCl

as the hydrochloride of (II):

To a solution of 0.781g of the above product in 15.6ml of dimethylformamide are added 1ml of 1.76 N dry hydrochloric acid/acetic acid and 1ml of palladium black, and the mixture is stirred under a hydrogen atmosphere and then worked up in the same manner as in Example (1) for preparing the starting material to give 0.666g ( in 95.97% yield) of the titled product, hexapeptide hydrazide hydrochloride [the hydrochloride of (II)].

What is claimed is:

A process for producing a caerulein intermediate decapeptide represented by the formula:

H-Pyr-Gln-Asp-Tyr-Thr(Ac)-Gly-Trp-Met-Asp-Phe-NH$_2$ (I)

which comprises subjecting the hexapeptide represented by the formula:

H-Pyr-Gln-Asp-Tyr-Thr(Ac)-Gly-NHNH$_2$ (II)

to azide forming reaction at the C-terminal and then to reaction with the tetrapeptide represented by the formula:

H-Trp-Met-Asp-Phe-NH$_2$ (III)

[wherein Ac means acetyl].